# EUROPÄISCHE PATENTSCHRIFT

(11) **EP 0 760 632 B1**
(45) Veröffentlichungstag und Bekanntmachung des Hinweises auf die Patenterteilung: **13.09.2000**
(21) Anmeldenummer: 95912118.7
(22) Anmeldetag: 27.03.1995
(51) Int. Cl.: A61B 17/80

(54) **KNOCHENPLATTE**
BONE PLATE
PLAQUE POUR OSTEOSYNTHESE

(43) Veröffentlichungstag der Anmeldung: 12.03.1997
(73) Patentinhaber: Synthes AG, Chur, 7002 Chur (CH)
(72) Erfinder: TALOS, Gilbert, CH-4515 Oberdorf (CH); SCHMOKER, Roland, CH-3012 Bern (CH)
(74) Vertreter: Lusuardi, Werther Giovanni, Dr.
(86) Internationale Anmeldenummer: CH9500065
(87) Internationale Veröffentlichungsnummer: WO9629948

(56) Entgegenhaltungen:
- EP-A- 0 053 999
- EP-A- 0 410 309
- WO-A-88/03781
- FR-A- 2 674 118

## Beschreibung

Die Erfindung bezieht sich auf eine Knochenplatte, gemäss dem Oberbegriff des Patentanspruchs 1.

Eine Knochenplatte, die die Merkmale des Oberbegriffs von Anspruch 1 aufweist, ist aus der EP-A-0 410 309 bekannt.

Aus der FR 742.618 ist eine Knochenplatte bekannt, bei welcher die kreisrunden Plattenbohrungen gegenüber der Plattennormalen geneigt sind und ein Innengewinde aufweisen. Sie gestatten die Aufnahme von Knochenschrauben mit einer zweiten - gegenüber der üblichen mit einem Gewinde versehenen Schraubenschaftpartie erweiterten - Gewindepartie, welche mit dem Innengewinde der Plattenbohrung korrespondiert. Durch die Neigung der Plattenbohrung ist es möglich die Knochenschrauben ebenfalls mit der von der Plattenbohrung vorgegeben Neigung im Knochen zu verankern.
Nachteilig bei dieser bekannten Knochenplatte ist der Umstand, dass die Neigung der Knochenschrauben nicht beliebig wählbar ist, sondern durch die Neigung der Plattenbohrung und des darin enthaltenen Innengewindes bereits vorgegeben ist.

Hier will die Erfindung Abhilfe schaffen. Der Erfindung liegt die Aufgabe zugrunde, eine Knochenplatte zu schaffen, deren Plattenbohrungen so beschaffen sind, dass wahlweise zwei verschiedene Typen von Schraubenverankerungen möglich sind.
Bei der ersten Applikationsart kann eine Knochenschraube mit sphärischem Kopf im Langloch der Platte innerhalb eines weiten wählbaren Bereiches mit einer Angulation gegenüber der Plattennormalen in den Knochen geschraubt werden. Durch eine Neigung der Plattenlochansenkung ist auch die Erzeugung einer Kompressionswirkung möglich.
Bei der zweiten Applikationsart kann eine Knochenschraube mit Gewindekopf als Pfeilerschraube im partiellen Innengewinde des Langlochs mit der Platte in rigider Weise vertikal verschraubt werden.

Die Erfindung löst die gestellte Aufgabe mit einer Knochenplatte, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass die erfindungsgemässe Knochenplatte für die vielfältigsten Anwendungsfälle (Fixation, Kompression, Verwendung als Fixateur interne im Sinne einer Pfeilerschraube) ubiquitär eingesetzt werden kann, insbesondere jedoch im maxillofazialen Bereich, wo es speziell auf die dreidimensionale Anpassbarkeit ankommt.

Weitere Vorteile der Erfindung sind die folgenden:
- Kompatibilität mit den üblichen Kortikalisschrauben;
- variable Angulationsmöglichkeit der Knochenschrauben im Plattenloch;
- problemlose Entfernbarkeit der Schrauben auch über einen intraoralen Zugang;
- Möglichkeit einer Reoperation mit Verwendung einer neuen Platte;
- Schraubenkopffixation im Plattenloch mit Knochenschrauben üblichen Durchmessers; und
- Ausübung einer Kompressionswirkung mittels Kugelkopfschrauben.

Die Erfindung und Weiterbildungen der Erfindung werden im folgenden anhand der teilweise schematischen Darstellungen eines Ausführungsbeispiels noch näher erläutert.

Es zeigen:
Fig. 1 eine perspektivische Teilansicht einer erfindungsgemässen Knochenplatte;
Fig. 2 eine partielle Aufsicht auf die Knochenplatte gemäss der Erfindung;
Fig. 3 einen partiellen Längsschnitt durch die Knochenplatte längs der Linie III-III von Fig. 2;
Fig. 4 einen zur Längsrichtung der Knochenplatte orthogonalen Profilquerschnitt längs der Linie IV-IV von Fig. 2;
Fig. 5 einen partiellen Längsschnitt durch eine erfindungsgemässe Knochenplatte mit senkrecht eingeschraubter Knochenschraube mit Doppel-Gewinde;
Fig. 6 einen partiellen Längsschnitt durch eine erfindungsgemässe Knochenplatte mit schräg oder gerade durchgeführter Knochenschraube mit sphärischem Kopf als Befestigungsschraube ohne Kompression;
Fig. 7 einen senkrecht zum Längsschnitt nach Fig. 6 stehenden Querschnitt im Lochbereich der Knochenplatte; und
Fig. 8 einen partiellen Längsschnitt durch eine erfindungsgemässe Knochenplatte mit gerade durchgeführter Knochenschraube mit sphärischem Kopf als Befestigungsschraube mit Kompression.

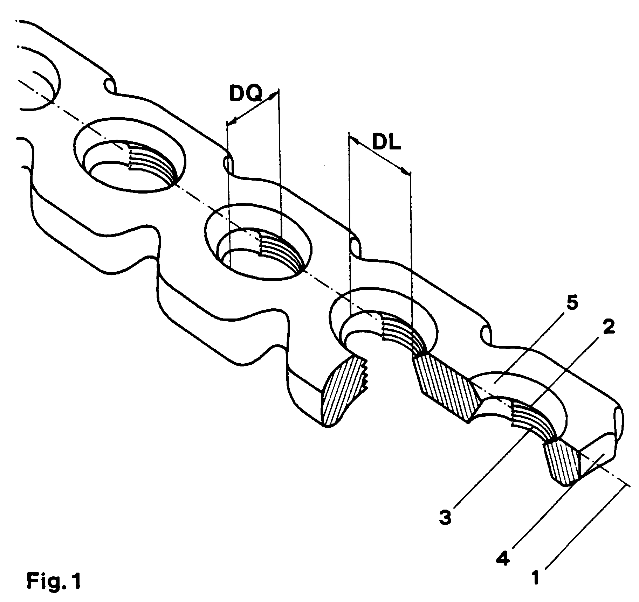

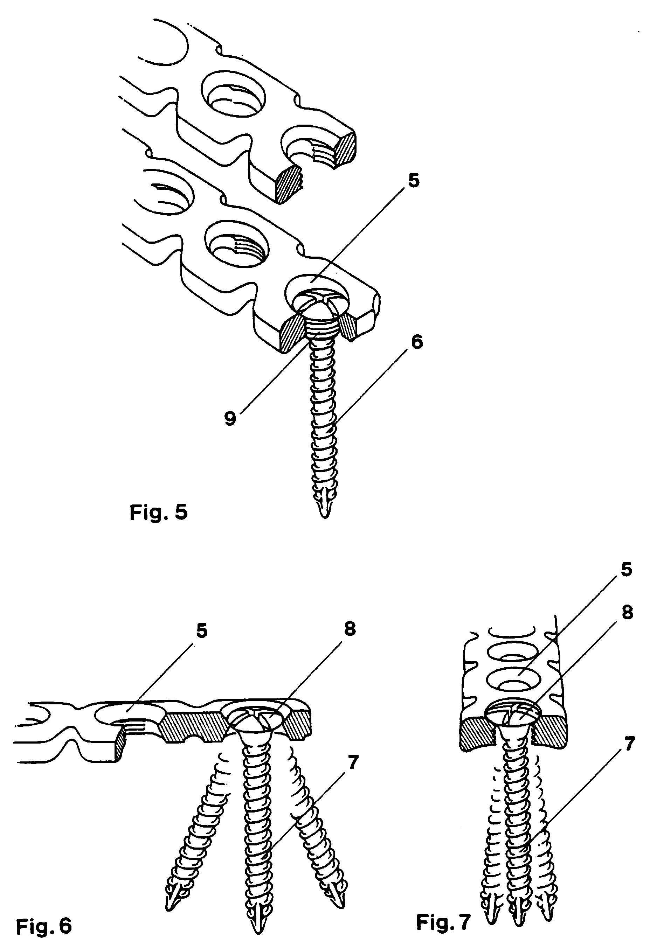

Die in Fig. 1 dargestellte Knochenplatte besitzt mehrere in Richtung der Plattenlängsachse 1 angeordnete Löcher 2 für die Aufnahme von (in den Fig. 5 und 6 dargestellten) verschiedenen Typen von Knochenschrauben 6 und 7, die zur Fixierung der Knochenplatte am Knochen bestimmt sind.

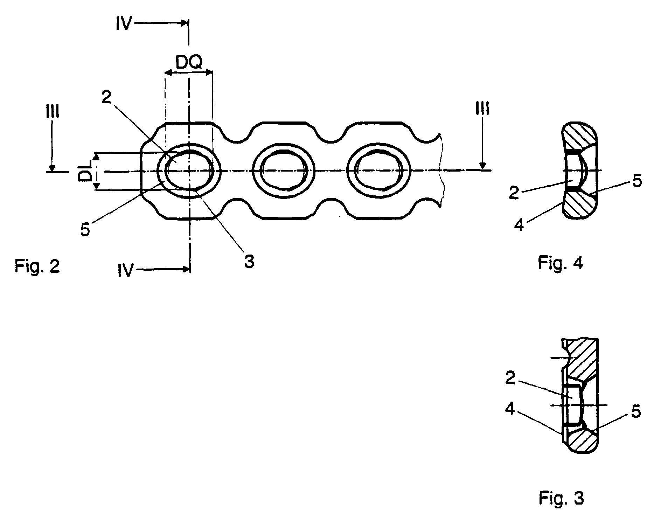

Die Löcher 2 sind, wie in den Fig. 2 - 4 im Detail dargestellt, als sogenannte Langlöcher ausgebildet, d.h. der Durchmesser D_{L} ist in Richtung der Plattenlängsachse 1 gemessen grösser als der senkrecht zur Plattenlängsachse 1 stehende Durchmesser D_{Q}.

Der untere, der Knochenapplikationsfläche 4 zugewandte Teil des Loches 2 ist - wie in Fig. 4 gezeigt - in der Querrichtung der Platte annähernd kreiszylindrisch ausgebildet und erweitert sich - wie in Fig. 3 gezeigt - in der Längsrichtung der Platte gegen die Knochenapplikationsfläche 4 hin annähernd konisch.
Im kreiszylindrischen Abschnitt des Loches 2 ist ein Innengewinde 3 angebracht, welches sich konstruktionsbedingt nur im Seitenbereich der Platte über einen Winkelbereich von je etwa 60° - 179°, vorzugsweise etwa 90° - 150° erstreckt.

Dieses partielle Innengewinde dient dazu eine - in Fig. 5 dargestellte - Knochenschraube 6 mit Gewindekopf 9 aufzunehmen. Durch Verschraubung des Aussengewindes der Gewindekopfes 9 mit dem korrespondierenden (partiellen) Innengewinde 3 ergibt sich eine rigide Verankerung zwischen Knochenschraube 6 und Platte. Eine derart verschraubte Knochenschraube 6 dient als Pfeilerschraube.

Der obere, der Knochenkontaktfläche 4 abgewandte Teil des Loches 2 ist oval ausgebildet und mit einer konischen Erweiterung 5 versehen, welche dazu dient eine - in den Fig. 6 und 7 dargestellte - Knochenschraube 7 mit einem kugeligen Kopf 8 gleitend aufzunehmen. Unter Knochenapplikationsfläche 4 ist dabei die im wesentlichen unmittelbar mit dem Knochen zu kontaktierende Fläche der Platte zu verstehen.

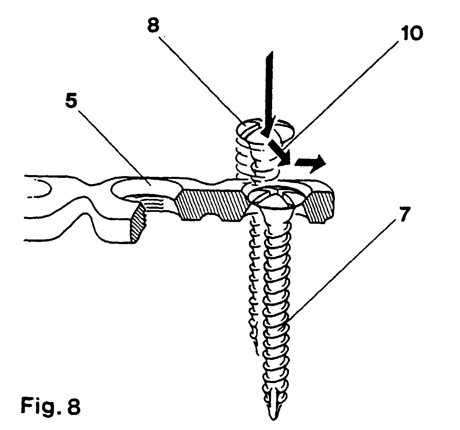

In Fig. 8 ist analog zu den Fig. 6 und 7 eine Knochenplatte mit einer Knochenschraube 7 mit einem kugeligen Kopf 8 dargestellt welche durch den Pfeil 10 angedeutet eine Kompressionswirkung ausüben kann. Dazu wird die konische Erweiterung 5 des Loches 2 - wie in Fig. 3 dargestellt - mit einem Kugel- oder Facetten-Fräser unter einem Winkel von 57° gegenüber der Plattenlängsachse 1 ausgeführt, so dass das entstehende Langloch für die Knochenschraube 7 wie eine Rampe funktioniert.

Diese spezielle Geometrie des Loches 2 gestattet die wahlweise Applikation von verschiedenen Typen von Knochenschrauben 6,7 mit ein und derselben Platte.

## Patentansprüche

1. Knochenplatte mit mehreren in Richtung der Plattenlängsachse (1) angeordneten Löchern (2) für die Aufnahme von Knochenschrauben, wobei
der Durchmesser D_{L} mindestens eines Loches (2) in Richtung der Plattenlängsachse (1) gemessen grösser ist als der Durchmesser D_{Q} dieses Loches (2) senkrecht zur Plattenlängsachse (1) gemessen,
dadurch gekennzeichnet, daß
mindestens eines dieser Löcher (2) mit einem kleineren Durchmesser D_{Q} senkrecht zur Plattenlängsachse (1) im Bereich dieses Durchmessers D_{Q} ein partielles Gewinde (3) zur Aufnahme einer Knochenschraube mit Gewindekopf aufweist.

2. Knochenplatte nach Anspruch 1, dadurch gekennzeichnet, dass mindestens eines der Löcher (2) mit einem kleineren Durchmesser D_{Q} in seinem oberen, der Knochenkontaktfläche (4) abgewandten Teil, eine konkave, vorzugsweise sphärische Erweiterung (5) zur Aufnahme einer Knochenschraube mit einem kugeligen Kopf aufweist.

3. Knochenplatte nach Anspruch 1 oder 2, dadurch gekennzeichnet, dass das Verhältnis zwischen D_{L}/D_{Q} im Bereich von 1,01 - 3,00, vorzugsweise von 1,1 - 1,5 liegt.

## Claims

1. A bone plate comprising several holes (2) arrayed in the direction of the plate longitudinal axis (1) and receiving bone screws, whereby the diameter D_{L} of at least one hole (2) when measured in the direction of the plate longitudinal axis (1) is larger than the diameter D_{Q} of this hole (2) when measured perpendicularly to the plate longitudinal axis (1),
characterized in that
at least one of these holes (2) comprising a lesser diameter D_{Q} perpendicularly to the plate longitudinal axis (1) evinces, in the area of this diameter D_{Q}, a partial thread (3) to seat a bone screw with a thread head.

2. Bone plate as claimed in claim 1, characterized in that at least one of the holes (2) comprising a lesser diameter D_{Q} in its upper part away from the bone contact surface (4) evinces a concave and preferably spherical flaring area (5) to receive a bone screw with a spherical head.

3. Bone plate as claimed in either of claims 1 and 2, characterized in that the ratio D_{L}/D_{Q} is within the range of 1,01 to 3,00 and preferably 1,1 to 1,5.

## Revendications

1. Plaque pour ostéosynthèse présentant plusieurs trous (2) disposés dans la direction de l'axe longitudinal (1) de la plaque, pour la réception de vis d'ostéosynthèse, le diamètre D_{L} d'au moins un trou (2), mesuré dans la direction de l'axe longitudinal (1) de la plaque, est supérieur au diamètre D_{Q} de ce trou (2), mesuré perpendiculairement à l'axe longitudinal (1) de la plaque,
caractérisée en ce que
au moins l'un de ces trous (2) dont le diamètre D_{Q} est plus petit dans le sens perpendiculaire à l'axe longitudinal (1) de la plaque présente dans la zone de ce diamètre D_{Q} un filet partiel (3) pour la réception d'une vis d'ostéosynthèse à tête filetée.

2. Plaque pour ostéosynthèse selon la revendication 1, caractérisée en ce qu'au moins l'un des trous (2) dont le diamètre D_{Q} est plus petit présente dans sa partie supérieure, non tournée vers la surface (4) de contact avec l'os, un évasement (5) concave, de préférence sphérique, pour la réception d'une vis d'ostéosynthèse à tête sphérique.

3. Plaque pour ostéosynthèse selon la revendication 1 ou 2, caractérisée en ce que le rapport D_{L}/D_{Q} est situé dans la plage de 1,0 - 3,00, de préférence de 1,1 -1,5.
